# EUROPÄISCHE PATENTSCHRIFT

(11) **EP 2 648 779 B1**
(45) Veröffentlichungstag und Bekanntmachung des Hinweises auf die Patenterteilung: **02.09.2020**
(21) Anmeldenummer: 11805404.8
(22) Anmeldetag: 07.12.2011
(51) Int. Cl.: A61M 5/142, F04B 43/12

(54) **PUMPENROTOR**
PUMP ROTOR
ROTOR DE POMPE

(30) Priorität: 09.12.2010 DE 102010053903; 09.12.2010 US 421288 P
(43) Veröffentlichungstag der Anmeldung: 16.10.2013
(73) Patentinhaber: Fresenius Medical Care Deutschland GmbH, 61352 Bad Homburg (DE)
(72) Erfinder: BRÜCKNER, Christoph, 97505 Geldersheim (DE)
(74) Vertreter: Laufhütte, Dieter
(86) Internationale Anmeldenummer: PCT/EP2011/006153
(87) Internationale Veröffentlichungsnummer: WO 2012/076173

(56) Entgegenhaltungen:
- WO-A1-95/17597
- FR-A1- 2 284 783
- US-A- 4 720 249
- US-A- 5 009 573
- US-A- 6 036 459

## Beschreibung

Die vorliegende Erfindung betrifft einen Pumpenrotor für eine Schlauchrollenpumpe, insbesondere eine medizinische Schlauchrollenpumpe. Des Weiteren betrifft die vorliegende Erfindung insbesondere eine medizinische Schlauchrollenpumpe sowie ein Verfahren zum Betätigen eines Pumpenrotors einer Schlauchrollenpumpe.

Aus dem Stand der Technik sind Schlauchrollenpumpen bekannt, bei denen die Rollen, die den eingelegten Schlauch okkludieren, federnd gelagert sind. Die Federkraft in Abhängigkeit der Compliance des eingelegten Schlauches bestimmt dabei den maximalen Nachpumpendruck, bei dem eine Okklusion der Pumpe noch gegeben ist.

Eine derartige Schlauchrollenpumpe ist dabei beispielsweise aus der DE 33 26 785 A1 bekannt. Demnach sind bei einem Pumpenrotor für peristaltisch arbeitende Rollenpumpen, insbesondere für Schlauchpumpen in der Medizintechnik, zwei Rollenträger mittels zweier Hebelstangen und zweier Federn derart getriebeverbunden und werden derart gesteuert, dass die auf einem Pumpenschlauch abrollenden Rollen einerseits auf einem Wälzkreis mit konstantem Durchmesser laufen und andeandererseits sich selbständig auf Pumpenschläuche mit unterschiedlichen Wandstärken oder Durchmessern einstellen können.

Im Fall einer Stenose stromabwärts der Pumpe wird so der Pumpendruck auf ungefährliche Werte begrenzt und verhindert, dass der Schlauch platzt oder ein nachfolgender Filter, wie z. B. ein Dialysefilter, Schaden nimmt. Auch kann hierdurch verhindert werden, dass beispielsweise in weiterer Folge ein Patient, dessen Blut beispielsweise im Rahmen einer Dialysebehandlung mit einer derartigen Schlauchrollenpumpe in einem extrakorporalen Blutkreislauf für die Dialyse gefördert wird, gefährdet wird.

Die erforderliche Federkonstante, mit der die Rollen an den eingelegten Schlauch angepresst werden, ist von der gewählten Behandlungsart, dem eingelegten Schlauch und dem nachfolgenden Filter abhängig und somit variabel.

Veränderte Anforderungen an die Federkraft, mittels derer die Rollen an den in die Schlauchrollenpumpe eingelegten Schlauch angedrückt werden, können bei den aus dem Stand der Technik bekannten Schlauchrollenpumpen nur durch einen Wechsel der Feder realisiert werden, was oftmals gleichbedeutend ist mit einem Austausch des Rotors. Dies ist jedoch vergleichsweise aufwändig. Darüber hinaus sind die Abstufungen der Federkraft in der Regel nicht beliebig klein, so dass nicht jede beliebige, jedoch ggf. erforderliche Anpassung der Okklusionskraft auch tatsächlich eingestellt werden kann.

Darüber hinaus muss bei der Auf- und Abrüstung der Pumpe mit dem Schlauch zusätzlich händisch Kraft aufgewendet werden, um die Rollen soweit zusammenzudrücken, dass der Schlauch auch ins Pumpenbett eingelegt werden kann.

Die Druckschrift US 6 036 459 A offenbart in den verschiedenen Ausführungsformen der peristaltischen Pumpe immer nur Betätigungsmittel, bei denen der Druck eines Mediums ausschließlich einseitig eines Kolbens oder innerhalb einer Expansionskammer wirkt.

Die US 4 720 249 A offenbart eine peristaltische Pumpe mit einer einseitig wirkenden hydraulischen Kolbenanordnung.

Die US 5 009 573 A zeigt eine Pumpe mit lediglich ein einfach wirkendes Betätigungsmittel, welches durch die Beaufschlagung mit Überdruck in die eine und durch die Beaufschlagung mit Unterdruck in die entgegengesetzte Richtung verfahrbar ist.

Die Druckschrift FR 2284 783 A1, welche eine Schlauchpumpe offenbart, geht in ihrer Offenbarung nicht über einen einseitig wirkenden Kolben hinaus, welches zum Füllen eines Zylinders mit ÖI und Druckluft dient.

Aus der WO 95/17597 A1 ist eine peristaltische Pumpe bekannt, die ein lineares Betätigungsmittel aufweist. Das lineare Betätigungsmittel wird pneumatisch aktiviert, wobei sich die Betätigerachse aufgrund des Aufbringens von pneumatischem Überdruck in die eine und aufgrund des pneumatischen Unterdrucks in die andere Richtung bewegt.

Bei all diesen Pumpen sind aber nur einseitig wirkende Betätigungsmittel vorgesehen, die mit Druck angetrieben werden.

Es ist daher die Aufgabe der vorliegenden Erfindung, einen Pumpenrotor sowie eine Schlauchrollenpumpe der eingangs genannten Art in vorteilhafter Weise weiterzubilden, insbesondere dahingehend, dass eine einfachere und schnellere Einstellung der auf einen in der Schlauchrollenpumpe eingelegten Schlauch erzeugbaren Anpress- und/oder Zusammendrückkraft der Schlauchrollenpumpe ermöglicht werden kann.

Diese Aufgabe wird erfindungsgemäß gelöst durch einen Pumpenrotor mit den Merkmalen des Anspruchs 1. Danach ist vorgesehen, dass ein Pumpenrotor für eine Schlauchrollenpumpe mit wenigstens einem Betätigungsmittel versehen ist, mittels dessen der Pumpenrotor hydraulisch und/oder pneumatisch betätigbar ist. Bei der Schlauchrollenpumpe kann es sich insbesondere um eine medizinische Schlauchrollenpumpe handeln, also um eine peristaltisch arbeitende Pumpe. Derartige Pumpen können insbesondere als Blutpumpen z. B. in einem extrakorporalen Blutkreislauf eines Dialysebehandlungssystems Verwendung finden. Grundsätzlich ist auch denkbar, derartige Schlauchrollenpumpen auf der Wasserseite bzw. im Wasserteil eines Dialysebehandlungssystems einzusetzen, also zur Förderung des Dialysates und/oder der Komponenten des Dialysates auf der Dialysatseite des Dialysebehandlungssystems.

Durch die Verwendung eines Betätigungsmittels, mittels dessen der Pumpenmotor hydraulisch und/oder pneumatisch betätigbar ist, ergibt sich insbesondere der Vorteil, dass der Grad an Einstellungsmöglichkeiten erhöht wird, insbesondere dadurch, dass durch das Betätigungsmittel eine stufenlose Betätigung aufgrund der hydraulischen und/oder pneumatischen Betätigung des Pumpenrotors möglich ist. Hierin liegt ein wesentlicher Unterschied und auch Vorteil gegenüber dem bisherigen Einsatz von Federn, wobei es jedoch grundsätzlich auch denkbar ist, zusätzlich zur hydraulischen und/oder pneumatischen Betätigung des Pumpenrotors auch weiterhin Federn einzusetzen.

Des Weiteren ist vorgesehen, dass der Pumpenrotor wenigstens eine Rotorrolle aufweist, die mittels des Betätigungsmittels einfahrbar und ausfahrbar ist. Es ist also möglich, die Rotorrolle in vorteilhafter Weise hydraulisch und/oder pneumatisch einfahren zu können, aber auch ausfahren zu können. Hierdurch wird es vorteilhaft ermöglicht, die Position der Rotorrolle hochgenau einstellen zu können.

Ferner ist möglich, dass durch das hydraulische und/oder pneumatische Verfahren des Pumpenrotors mittels der Rotorrolle auf einen in der Schlauchrollenpumpe eingelegten Schlauch eine Anpress- und/oder Zusammendrückkraft erzeugbar und/oder einstellbar ist. Da mittels der vorteilhaften stufenlosen und hochgenauen Positionierbarkeit der Rotorrolle mittels des hydraulischen und/oder pneumatischen Betätigungsmittels auch die Position von Rotorrolle relativ zum Schlauchpumpenbett einstellbar ist, kann hierdurch die Okklusion hochgenau eingestellt werden. Die auf den in der Schlauchrollenpumpe eingelegten Schlauch erzeugbare Anpress- und/oder Zusammendrückkraft mittels der Rotorrolle ist somit variabel und stufenlos einstellbar.

Die auf den in der Schlauchrollenpumpe eingelegten Schlauch wirkende Anpress- und/oder Zusammendrückkraft kann auch Okklusionskraft genannt werden.

Insbesondere kann vorgesehen sein, dass wenigstens ein Steuerungs- und/oder Regelungsmittel vorgesehen ist und dass die Anpress- und/oder Zusammendrücckraft variabel und/oder prozessgesteuert und/oder prozessgeregelt mittels des Steuerungs- und/oder Regelungsmittels steuer- und/oder regelbar ist, insbesondere unter Einbeziehung der Faktoren von in die Schlauchrollenpumpe eingesetztem Schlauch, Betriebs- und Einsatzart der Schlauchrollenpumpe und/oder eingesetzten Komponenten wie einem Dialysefilter als Teil eines extrakorporalen Blutkreislaufs, der mit der Schlauchrollenpumpe betrieben wird. Es ist also nunmehr vorteilhaft möglich, situationsgerecht und zeitnah, ohne dass es eines teilweisen Zerlegens der Schlauchrollenpumpe bedarf, auf veränderte Anforderungen hinsichtlich der vorgenannten Anpress- und/oder Zusammendrückkraft zu reagieren. Dies kann nunmehr in vorteilhafter Ausgestaltung halbautomatisch oder automatisch mittels des Steuerungs- und/oder Regelungsmittels erfolgen. So können bei einer automatischen Steuerung- und/oder Regelung anhand vordefinierter Vorgaben Anpassungen der Anpress- und/oder Zusammendrückkraft vorgenommen werden. Bei einer halbautomatischen Steuerung- und/oder Regelung ist es beispielsweise denkbar, dass alternativ und/oder zusätzlich Eingaben der Bediener der Schlauchrollenpumpe Berücksichtigung finden können.

Des Weiteren ist es möglich, dass das Betätigungsmittel ein bzw. einen Pneumatik- und/oder ein Hydraulikaktuator, besonders bevorzugt ein bzw. einen Pneumatik- und/oder ein Hydraulikzylinder ist und/oder umfasst. Es kann dabei vorgesehen sein, dass die Rotorrolle mittelbar und/oder unmittelbar am Kolben des Pneumatik- und/oder Hydraulikzylinders befestigt bzw. gelagert ist. Genauso gut kann es aber auch vorgesehen sein, dass die Rotorrolle am Pneumatik- und/oder Hydraulikzylinder selbst mittelbar und/oder unmittelbar befestigt ist, und dass der Kolben des Pneumatik- und/oder Hydraulikzylinders mittelbar und/oder unmittelbar am Pumpenrotor befestigt ist.

Gemäß der Erfindung ist vorgesehen, dass das Betätigungsmittel ein doppelt wirkendes Betätigungsmittel ist, wobei das doppelt wirkende Betätigungsmittel vorzugsweise ein bzw. einen doppelt wirkender Hydraulikzylinder bzw. Hydraulikkolben ist und/oder umfasst, der aktiv in eine erste und in zweite Richtung bewegbar ist, insbesondere mittels eines hydraulisch und/oder pneumatisch erzeugten Überdruckes aktiv in eine erste und in zweite Richtung bewegbar ist. In dieser vorteilhaften Ausführungsform werden sogenannte doppelt wirkende Kolben verwendet, die beidseitig des Kolbens mit einem hydraulischen und/oder pneumatisch erzeugten Druck beaufschlagt werden können. Somit kann der Kolben aktiv in beide Richtungen durch entsprechende Ventilsteuerungsmittel bewegt werden. Diese Ausführung hat beispielsweise den Vorteil, dass nur ein Überdruck benötigt wird. Bei einer pneumatischen Ausführung ergibt sich dabei insbesondere der Vorteil, dass hierbei der bei einer übergeordneten Maschine, wie z. B. einer Dialysemaschine, ohnehin zumeist vorhandene Luftüberdruck ohne Weiteres verwendet werden kann.

Nicht erfindungsgemäß ist es möglich, dass das Betätigungsmittel ein mittels Überdruck und Unterdruck betätigbares Betätigungsmittel ist, wobei das Betätigungsmittel mittels Überdruck in eine erste Richtung bewegbar ist und mittels Unterdruck in eine zweite Richtung bewegbar ist. So kann vorteilhafterweise bei einem vorhandenen Unterdruck bzw. einer vorhandenen Möglichkeit zur Erzeugung eines Unterdruckes eine Vor- und Zurückbewegung des Pumpenrotors auch durch ein Anlegen eines Über- und/oder Unterdrucks einseitig des Kolbens realisiert werden.

Durch die nun mögliche aktive Zurückbewegung der Rollen der Schlauchrollenpumpe, die an dem Pumpenrotor angeordnet und gelagert sind, wobei die aktive Verfahrbarkeit der Rollen mittels Überdruck oder durch eine Kombination von Überdruck- und Unterdruck erreicht wird, wird nunmehr auch das Einlegen des Schlauches in das Pumpenbett der Schlauchrollenpumpe erheblich erleichtert.

Ferner betrifft die vorliegende Erfindung eine Schlauchrollenpumpe mit den Merkmalen des Anspruchs 3. Danach ist vorgesehen, dass eine Schlauchrollenpumpe mit wenigstens einem Pumpenrotor nach Anspruch 1 oder 2 versehen ist.

Außerdem betrifft die vorliegende Erfindung eine Blutbehandlungsmaschine mit den Merkmalen des Anspruchs 4. Danach ist vorgesehen, dass eine Blutbehandlungsmaschine mit wenigstens einem Pumpenrotor nach Anspruch 1 oder 2 und/oder mit wenigstens einer Schlauchrollenpumpe nach Anspruch 3 versehen ist, wobei die Blutbehandlungsmaschine vorzugsweise eine Dialysemaschine ist.

Darüber hinaus betrifft die vorliegende Erfindung ein Verfahren zum Betätigen eines Pumpenrotors einer Schlauchrollenpumpe mit den Merkmalen des Anspruchs

5. Dabei ist vorgesehen, dass bei einem Verfahren zum Betätigen eines Pumpenrotors einer Schlauchrollenpumpe, insbesondere zum Einstellen des Okklusionsdruckes und/oder der Position der Rotorrollen einer Schlauchrollenpumpe, der Pumpenrotor der Schlauchrollenpumpe hydraulisch und/oder pneumatisch betätigt wird. Dabei ist die Kraft, die auf den Pumpenschlauch wirkt, eine Größe, die den Flüssigkeitsdruck flussabwärts der Pumpe begrenzt. Der somit maximal mögliche Flüssigkeitsdruck flussabwärts der Pumpe wird auch Okklusionsdruck genannt. Der Okklusionsdruck ist eine sicherheitsrelevante Größe bei der Auslegung von z. B. Blutpumpen für die Dialyse. Kommt es z. B. durch eine Zusetzung eines Dialysefilters, der sich flussabwärts der Blutpumpe in einem Hämodialysegerät befindet, zu einer Stenose flussabwärts der Blutpumpe, steigt der Flusswiderstand für das Blut zwischen der Blutpumpe und dem Dialysefilter stark an. Durch die okkludierende Förderung der als Schlauchrollenpumpe ausgeführten Blutpumpe steigt in Folge dessen auch der Flüssigkeitsdruck flussabwärts der Blutpumpe stark an, wodurch es zu einer Zerstörung der Blutkörperchen, also zu einer Hämolyse kommen kann. Darüber hinaus können zu hohe Flüssigkeitsdrücke flussabwärts der Blutpumpe zur Zerstörung des Blutschlauches oder der Hohlfasern des Dialysefilters führen, was die Gesundheit des Patienten gefährdet. Es ist daher sicherheitsrelevant, den Flüssigkeitsdruck flussabwärts der Blutpumpe zu begrenzen.

Des Weiteren ist vorgesehen, dass der Pumpenrotor wenigstens eine Rotorrolle aufweist, die hydraulisch und/oder pneumatisch eingefahren und ausgefahren werden kann.

Außerdem ist vorgesehen, dass durch das hydraulische und/oder pneumatische Verfahren des Pumpenrotors mittels der Rotorrolle auf einen in der Schlauchrollenpumpe eingelegten Schlauch eine Anpress- und/oder Zusammendrückkraft erzeugt wird.

Insbesondere ist denkbar, dass die Anpress- und/oder Zusammendrückkraft variabel und/oder prozessgesteuert und/oder prozessgeregelt gesteuert und/oder geregelt wird, insbesondere unter Einbeziehung von in die Schlauchrollenpumpe eingesetztem Schlauch, Betriebs- und Einsatzart der Schlauchrollenpumpe und/oder eingesetzten Komponenten wie einem Dialysefilter als Teil eines extrakorporalen Blutkreislaufs, der mit der Schlauchrollenpumpe betrieben wird.

Erfindungsgemäß ist weiterhin vorgesehen, dass das hydraulische und/oder pneumatische Verfahren des Pumpenrotors in eine erste und in zweite Richtung aktiv mittels hydraulisch und/oder pneumatisch erzeugtem Überdruck erfolgt.

Außerdem ist denkbar, dass das hydraulische und/oder pneumatische Verfahren des Pumpenrotors in eine erste und in eine zweite Richtung mittels Überdruck in eine erste Richtung erfolgt und mittels Unterdruck in eine zweite Richtung erfolgt. Die erste Richtung kann dabei die Ausfahrrichtung sein und die zweite Richtung kann dabei die Einfahrrichtung sein.

Ferner kann vorgesehen sein, dass das Verfahren unter Einsatz wenigstens eines Pumpenrotors nach einem der Ansprüche 1 oder 2 und/oder wenigstens einer Schlauchrollenpumpe nach Anspruch 3 und/oder wenigstens einer Blutbehandlungsmaschine nach Anspruch 4 durchgeführt wird.

Darüber hinaus betrifft die vorliegende Erfindung die Verwendung eines Pneumatik- und/oder eines Hydraulikaktuators zur Einstellung des Okklusionsdruckes einer Schlauchrollenpumpe mit den Merkmalen des Anspruchs 8. Danach ist vorgesehen, dass ein Pneumatik- und/oder Hydraulikaktuator zur Einstellung des Okklusionsdruckes einer Schlauchrollenpumpe verwendet wird, wobei der Pneumatik- und/oder Hydraulikaktuator vorzugsweise ein Betätigungsmittel nach Anspruch 7 ist und/oder wobei die Schlauchrollenpumpe vorzugsweise eine Schlauchrollenpumpe mit den Merkmalen des Anspruchs 3 ist

Weitere Einzelheiten und Vorteile der Erfindung sollen nun anhand eines in den Figuren dargestellten Ausführungsbeispiels näher erläutert werden.

### Es zeigen:

- Figur 1:: ein Ausführungsbeispiel der erfindungsgemäßen Schlauchrollenpumpe mit eingefahrenem Rotor; und
- Figur 2:: die erfindungsgemäße Schlauchrollenpumpe mit ausgefahrenem Rotor.

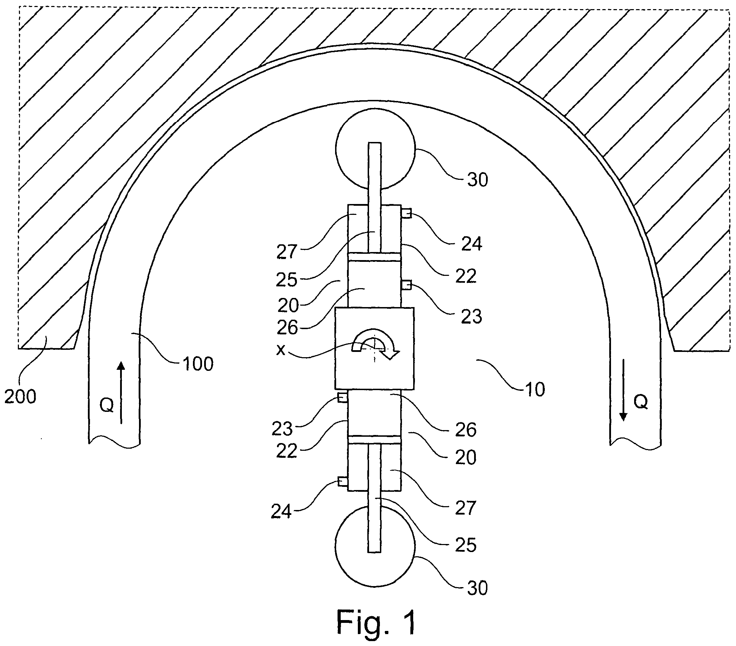

In Figur 1 ist ein Pumpenrotor 10 einer Schlauchrollenpumpe für medizinische Anwendungen gezeigt. Derartige medizinische Anwendungen sind insbesondere Dialysebehandlungen, wobei eine derartige Schlauchrollenpumpe vorzugsweise Bestandteil eines Dialysebehandlungsgeräts ist. Hierbei ist das Dialysebehandlungsgerät vorzugsweise eine Dialysemaschine, die zur Ausführung von Hämodialyse, Hämofiltration oder Hämodiafiltration eingerichtet sein kann. Dem Fachmann ist klar, dass der erfindungsgemäße Pumpenrotor 10 einer Schlauchrollenpumpe ohne Einschränkung bei jedem Gerät anwendbar ist, bei der eine Flüssigkeit durch eine Schlauchrollenpumpe gefördert wird. Beispiele hierfür sind Geräte zur automatischen Peritonealdialyse, Vorrichtungen zur Plasmapherese, Infusionsapparate oder Ähnliches. Die geförderte Flüssigkeit ist bevorzugt Blut, kann aber jedwede Flüssigkeit, insbesondere jedwede medizinische Flüssigkeit sein, wie z. B. Dialysierflüssigkeit oder Infusionsflüssigkeiten.

Die Schlauchrollenpumpe weist ein Pumpenbett 200 auf, in das der Schlauch 100 eingelegt ist, wobei das in dem Schlauch 100 geförderte Fluid in Förderrichtung Q gefördert wird.

Dies ist in Figur 1 bereits entsprechend eingezeichnet, wobei Figur 1 allerdings eine Situation zeigt, bei der die Rotorrolle 30 den Schlauch 100 noch nicht berührt. Dies entspricht der Situation des Aufrüstens oder Abrüstens der Schlauchrollenpumpe. Wie dies anhand von Figur 1 einfach nachvollziehbar und ersichtlich wird, ist es mittels der Schlauchrollenpumpe mit dem Pumpenrotor 10 ohne Weiteres und sehr einfach möglich, das Aufrüsten und/oder Abrüsten erheblich zu erleichtern. Denn hierzu kann bei der Durchführung des Verfahrens zum Betätigen eines Pumpenrotors vorgesehen sein, dass automatisch oder halbautomatisch, etwa auf Eingabe des Nutzers hin, ein Einfahren der Rotorrolle 30 zum erleichterten Einlegen des Schlauches 100 in des Pumpenbett 200, gefolgt von einem Ausfahren der Rotorrolle 30, im Zuge des Aufrüstens vorgenommen wird. Dies kann beim Abrüsten entsprechend erfolgen, nämlich derart, dass ein Einfahren der Rotorrolle 30 zum erleichterten Herausnehmen des Schlauches 100 aus dem Pumpenbett 200, ggf. gefolgt von einem zumindest teilweisen, erneuten Ausfahren der Rotorrolle 30 in eine sogenannte Parkposition, im Zuge des Abrüstens vorgenommen wird. Es kann auch vorgesehen sein, dass im Zuge des Abrüstens nur ein automatisches oder halbautomatisches Einfahren der Rotorrolle 30 erfolgt, um den Schlauch 100 im Pumpenbett 200 freizugeben und ein erleichtertes Entnehmen des Schlauches 100 zu ermöglichen.

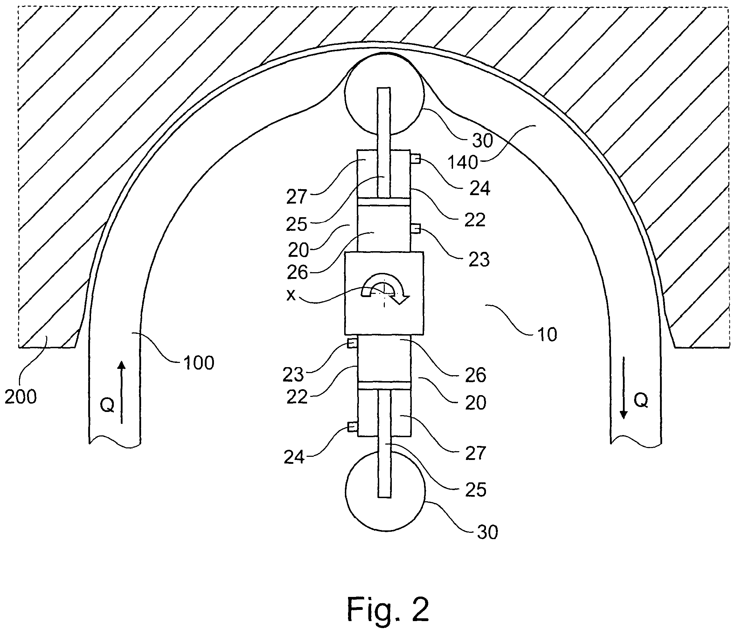

Der Pumpenrotor 10 rotiert zur Förderung des Fluids, insbesondere des im Schlauch 100 befindlichen Blutes in Förderrichtung Q, um die Drehachse x, hier im Uhrzeigersinn rotierend dargestellt, und drückt mit den Rotorrollen 30 den in die Schlauchrollenpumpe eingelegten Schlauch 100 so gegen das Pumpenbett 200 der Schlauchrollenpumpe, dass der Schlauch im Bereich der Rotorrollen okkludiert, wie dies in Figur 2 gezeigt ist. Durch die Drehbewegung des Pumpenrotors 10 erfolgt eine Förderung des Fluids 100 in Förderrichtung Q.

Der zweiarmige Pumpenrotor 10 mit zwei Rotorrollen 30 weist hier gemäß einem Ausführungsbeispiel der Erfindung zwei Betätigungsmittel 20 auf, mittels derer jeweils eine Rotorrolle 30 pneumatisch betätigbar ist.

Grundsätzlich kann auch vorgesehen sein, dass es sich bei dem Betätigungsmittel 20 um einen Hydraulikzylinder 22 handelt, mittels dessen die Rotorrollen 30 hydraulisch ein- und ausgeschoben werden können.

Bei dem in Figur 1 und 2 gezeigten Ausführungsbeispiel handelt es sich jedoch bei den Betätigungsmitteln 20, mittels derer jeweils die Rotorrolle 30 ein- und ausgeschoben werden kann, um Pneumatikzylinder 22, die über die Anschlüsse 23 und 24 mit entsprechenden Versorgungsleitungen verbunden sind.

Die Anschlüsse 23 und 24 können auch mit einer entsprechenden Ventilsteuerung in Verbindung stehen.

Mit einer derartigen pneumatischen Ventilsteuerung, die in Figur 1 und 2 nicht näher dargestellt ist, ist es beispielsweise möglich, bei einem als doppelt wirkend ausgeführten Pneumatikkolben, den Kolben 25 zu beiden Seiten mit Druckluft beaufschlagen zu können. Beispielsweise kann bei Beaufschlagung des Kolbens 25 über die Kammer 26 bzw. den Kolbenraum 26, der über den Anschluss 23 mit einer Überdruckleitung verbunden ist, der Kolben 25 ausgeschoben werden. Hierdurch wird die Rotorrolle 30 stärker gegen den Schlauch 100 und das Pumpenbett 200 gepresst, wodurch die Okklusionskraft erhöht wird. Außerdem ist es möglich, den Kolben 25 durch Beaufschlagung des Kolbens über die an dem Anschluss 24 angeschlossene Überdruckleitung und Erhöhung des Drucks im Ringraum 27 wieder einzuziehen und hierdurch die Okklusionskraft zu verringern bzw. die Rotorrolle 30 etwa für ein erleichtertes Einlegen des Schlauches zurückzuziehen. Durch einen derartigen doppelt wirkenden Kolben kann also der Kolben 25 aktiv in beide Richtungen, also die Einfahr- und Ausfahrrichtung mit Druckluft beaufschlagt werden. Der benötigte Luftüberdruck wird vorteilhafterweise durch die übergeordnete Maschine, hier eine Dialysemaschine, bereitgestellt.

Durch die Beaufschlagung eines bestimmten Überdruckes im Kolbenraum 26 gegenüber dem Ringraum 27 wird die Rotorrolle 30 mit einer bestimmten Kraft gegen den Schlauch 100 verfahren, wodurch dieser okkludierend an das Pumpenbett 200 gepresst wird. Dies ist in Figur 2 gezeigt, wobei ergänzend hinzuzufügen ist, dass der Schlauch 100 eine nicht näher dargestellte Wandstärke aufweist, was in der Figur 2 , die insoweit nur eine schematische Darstellung zeigt, nicht näher berücksichtigt ist.

Der Schlauchabschnitt 140 befindet sich flussabwärts der Rotorrolle 30 und ist beispielsweise mit einem in der Figur 1 oder 2 nicht gezeigten Dialysefilter verbunden. Der Flüssigkeitsdruck in diesem Schlauchabschnitt 140 ist abhängig von dem Flusswiderstand, der sich hier einstellt, und wirkt der Kraft, mit der die Rotorrolle 30 den Schlauch 100 an das Pumpenbett 200 presst, entgegen. Zusätzlich wirkt die Rückstellkraft des Schlauches 100, die in erster Linie von dessen Materialeigenschaften abhängt, der Kraft, mit der die Rotorrolle 30 den Schlauch 100 an das Pumpenbett 200 presst, entgegen. Die Anpresskraft der Rotorrolle 30 ist entsprechend so zu bemessen, dass im Normalbetrieb die Kraft ausreicht, um diese Gegenkräfte zu überwinden und den Schlauch 100 an das Pumpenbett 200 okkludierend anzupressen. Kommt es zu einer übermäßigen Erhöhung des Flusswiderstands im Schlauchabschnitt 140, steigt der Flüssigkeitsdruck in diesem Schlauchabschnitt 140 schnell an, wodurch die Gegenkraft, die entgegen der Kraft, mit der die Rotorrolle 30 den Schlauch 100 an das Pumpenbett 200 presst, wirkt, größer wird. Übersteigt die Gegenkraft die Anpresskraft der Rotorrolle 30, wird die Rotorrolle 30 zurück gedrückt, wodurch die Okklusion des Schlauches 100 aufgehoben wird. Hierdurch wird der Flüssigkeitsdruck im Schlauchabschnitt 140 begrenzt.

Als alternative Ausführungsform ist auch denkbar, dass über den Anschluss 23 eine Beaufschlagung des Kolbens 25 mit Überdruck, aber auch Unterdruck realisiert wird. Der Anschluss 24 kann somit entfallen oder dient somit nur als Anschluss an ein entsprechendes Ausgleichsreservoir oder als für Wartungszwecke oder Sicherheitszwecke bereitgestellter Anschluss 24.

Als Steuerungs- und/oder Regelungsmittel kann eine Steuerungs- und/oder Regelungseinheit vorgesehen sein, die in vorteilhafter Ausgestaltung Bestandteil der Steuerungs- und/oder Regelungseinheit der Dialysemaschine ist. Mittels der Steuerungs- und/oder Regelungseinheit kann die Okklusionskraft stufenlos und variabel, insbesondere bedarfsgerecht gesteuert bzw. eingeregelt werden. Insbesondere kann die Anpress- und/oder Zusammendrückkraft bzw. die Okklusionskraft über die Pneumatik variabel und prozessgesteuert bzw. prozessgeregelt eingestellt werden. Hierbei können auch Randvariablen wie die Beschaffenheit des Schlauches 100, die gewählte Behandlungsart oder ein eingesetzter Filter berücksichtigt werden. Sollte durch die Steuerungs- und/oder Regelungseinheit beispielsweise in Folge eines abrupten Druckanstieges, wie es beispielsweise bei einer Stenose stromabwärts der Pumpe der Fall ist, eine gefährliche Situation für den Patienten anhand beispielsweise im Speicher der Steuerungs- und/oder Regelungseinheit abgelegter Schwellwerte maschinenseitig erkannt werden, so wird die Rotorrolle 30 bzw. so werden die Rotorrollen 30 derart mittels des Pneumatikzylinders 22 positioniert, so dass der Pumpendruck auf ungefährliche Werte begrenzt und somit verhindert wird, dass der in die Schlauchrollenpumpe eingelegte Schlauch 100 platzt oder ein nachfolgender Filter, wie z. B. der Dialysefilter Schaden nimmt. Auch kann hierdurch verhindert werden, dass es zu einer Hämolyse des Blutes des Patienten kommt, da der Pumpendruck umgehend auf ungefährliche Werte begrenzt werden kann.

Mittels der Steuerungs- und/oder Regelungseinheit kann ferner für den Arbeitsschritt Einlegen des Schlauches 100 in die Schlauchrollenpumpe bzw. Entfernen des Schlauches 100 aus der Schlauchrollenpumpe automatisch vorgesehen sein, dass der Kolben 25 mit der daran befestigten Rotorrolle 30 jeweils vollständig eingefahren wird, so dass das Einlegen bzw. Entnehmen des Schlauches 100 in die bzw. aus der Schlauchrollenpumpe vorteilhaft vereinfacht ist.

## Patentansprüche

1. Pumpenrotor (10) für eine Schlauchrollenpumpe, insbesondere eine medizinische Schlauchrollenpumpe, mit wenigstens einem Betätigungsmittel (20), mittels dessen der Pumpenrotor (10) hydraulisch und/oder pneumatisch betätigbar ist, wobei der Pumpenrotor (10) wenigstens eine Rotorrolle (30) aufweist, die mittels des Betätigungsmittels (20) einfahrbar und ausfahrbar ist, wobei durch das hydraulische und/oder pneumatische Verfahren des Pumpenrotors (10) mittels der Rotorrolle (30) auf einen in der Schlauchrollenpumpe eingelegten Schlauch eine Anpress- und/oder Zusammendrückkraft erzeugbar und/oder einstellbar ist,
**dadurch gekennzeichnet,**
**dass** das Betätigungsmittel (20) ein bzw. einen doppelt wirkender/n Hydraulik- und/oder Pneumatikzylinder (22) bzw. Hydraulik- und/oder Pneumatikkolben (22) ist oder umfasst, der mittels eines hydraulisch und/oder pneumatisch erzeugten Überdruckes aktiv in eine erste und in eine zweite Richtung bewegbar ist.

2. Pumpenrotor (10) nach Anspruch 1, **dadurch gekennzeichnet, dass** wenigstens ein Steuerungs- und/oder Regelungsmittel vorgesehen ist und dass die Anpress- und/oder Zusammendrückkraft variabel und/oder prozessgesteuert und/oder prozessgeregelt mittels des Steuerungs- und/oder Regelungsmittels steuer- und/oder regelbar ist, insbesondere unter Einbeziehung der Faktoren von in die Schlauchrollenpumpe eingesetztem Schlauch, Betriebs- und Einsatzart der Schlauchrollenpumpe und/oder eingesetzten Komponenten wie einem Dialysefilter als Teil eines extrakorporalen Blutkreislaufs, der mit der Schlauchrollenpumpe betrieben wird.

3. Schlauchrollenpumpe mit wenigstens einem Pumpenrotor (10) nach Anspruch 1 oder 2.

4. Blutbehandlungsmaschine mit wenigstens einem Pumpenrotor (10) nach Anspruch 1 oder 2 und/oder mit wenigstens einer Schlauchrollenpumpe nach Anspruch 3, wobei die Blutbehandlungsmaschine vorzugsweise eine Dialysemaschine ist.

5. Verfahren zum Betätigen eines Pumpenrotors (10) einer Schlauchrollenpumpe, insbesondere zum Einstellen des Okklusionsdruckes und/oder der Position der Rotorrollen der Schlauchrollenpumpe, wobei hydraulisch und/oder pneumatisch der Pumpenrotor (10) betätigt wird, wobei der Pumpenrotor (10) wenigstens eine Rotorrolle (30) aufweist, die hydraulisch und/oder pneumatisch eingefahren und/oder ausgefahren werden kann, wobei durch das hydraulische und/oder pneumatische Verfahren des Pumpenrotors (10) mittels der Rotorrolle (30) auf einen in der Schlauchrollenpumpe eingelegten Schlauch eine Anpress- und/oder Zusammendrückkraft erzeugt wird, **dadurch gekennzeichnet,**
**dass** das hydraulische und/oder pneumatische Verfahren des Pumpenrotors (10) in eine erste und in eine zweite Richtung aktiv mittels hydraulisch und/oder pneumatisch erzeugtem Überdruck und einem doppelt wirkenden Hydraulik- und/oder Pneumatikzylinder (22) bzw. Hydraulik- und/oder Pneumatikkolben (22) erfolgt.

6. Verfahren nach Anspruch 5, **dadurch gekennzeichnet, dass** die Anpress- und/oder Zusammendrückkraft variabel und/oder prozessgesteuert und/oder prozessgeregelt gesteuert und/oder geregelt wird, insbesondere unter Einbeziehung von in die Schlauchrollenpumpe eingesetztem Schlauch, Betriebs- und Einsatzart der Schlauchrollenpumpe und/oder eingesetzten Komponenten wie einem Dialysefilter als Teil eines extrakorporalen Blutkreislaufs, der mit der Schlauchrollenpumpe betrieben wird.

7. Verfahren nach einem der Ansprüche 5 oder 6, **dadurch gekennzeichnet, dass** das Verfahren unter Einsatz wenigstens eines Pumpenrotors (10) nach einem der Ansprüche 1 oder 2 und/oder wenigstens einer Schlauchrollenpumpe nach Anspruch 3 und/oder wenigstens einer Blutbehandlungsmaschine nach Anspruch 4 durchgeführt wird.

8. Verwendung eines Pneumatik- und/oder eines Hydraulikaktuators (22) zur Einstellung des Okklusionsdruckes einer Schlauchrollenpumpe, wobei die Schlauchrollenpumpe eine Schlauchrollenpumpe mit den Merkmalen des Anspruchs 3 ist.

## Claims

1. Pump rotor (10) for a peristaltic hose pump, in particular a medical peristaltic hose pump, with at least one actuating means (20), by means of which the pump rotor (10) can be actuated hydraulically and/or pneumatically, wherein the pump rotor (10) includes at least one rotor roller (30) which can be retracted and/or extended, by means of the actuating means (20), wherein by hydraulically and/or pneumatically moving the pump rotor (10) by means of the rotor roller (30) onto a hose inserted into the peristaltic hose pump a pressing and/or compression force can be generated and/or be adjusted,
**characterized in that**
the actuating means (20), is and/or comprises a double-acting hydraulic and/or pneumatic cylinder (22) or hydraulic and/or pneumatic piston (22),
which is actively movable in a first and in a second direction by means of a hydraulically and/or pneumatically generated positive pressure.

2. Pump rotor (10) according to Claim 1, **characterized in that** at least one control and/or regulating means is provided and that the pressing and/or compression force can be controlled and/or regulated in a variable and/or process-controlled and/or process-regulated manner by means of the control and/or regulating means, in particular by taking into account the factors of the hose inserted into the peristaltic hose pump, of the type of operation and use of the peristaltic hose pump and/or of the used components such as a dialysis filter as part of an extracorporeal blood circuit, which is operated with the peristaltic hose pump.

3. Peristaltic hose pump with at least one pump rotor (10) according to Claim 1 or 2

4. Blood treatment machine with at least one pump rotor (10) according to Claim 1 or 2 and/or with at least one peristaltic hose pump according to Claim 3, wherein the blood treatment machine preferably is a dialysis machine.

5. Method for actuating a pump rotor (10) of a peristaltic hose pump, in particular for adjusting the occlusion pressure and/or the position of the rotor rollers of a peristaltic hose pump, wherein the pump rotor (10) is actuated hydraulically and/or pneumatically, wherein the pump rotor (10) comprises at least one rotor roller (30) which can be retracted and/or extended hydraulically and/or pneumatically, wherein by hydraulically and/or pneumatically moving the pump rotor (10) by means of the rotor roller (30) onto a hose inserted into the peristaltic hose pump a pressing and/or compression force is generated,
**characterized in that**
hydraulically and/or pneumatically moving the pump rotor (10) in a first and in a second direction is actively effected by means of hydraulically and/or pneumatically generated positive pressure and a double-acting hydraulic and/or pneumatic cylinder (22) or hydraulic and/or pneumatic piston (22).

6. Method according to claim 5, **characterized in that** the pressing and/or compression force is controlled and/or regulated in a variable and/or process-controlled and/or process-regulated manner, in particular by taking into account the hose inserted into the peristaltic hose pump, the type of operation and use of the peristaltic hose pump and/or the used components such as a dialysis filter as part of an extracorporeal blood circuit, which is operated with the peristaltic hose pump.

7. Method according to one of claims 5 or 6, **characterized in that** the method is performed by using at least one pump rotor (10) according to any of claims 1 or 2 and/or at least one peristaltic hose pump according to claim 3 and/or at least one blood treatment machine according to claim 4.

8. Use of a pneumatic and/or hydraulic actuator (22) for adjusting the occlusion pressure of a peristaltic hose pump, wherein the peristaltic hose pump is a peristaltic hose pump with the features of claim 3.

## Revendications

1. Rotor de pompe (10) pour une pompe péristaltique, en particulier une pompe péristaltique médicale, comprenant au moins un moyen d'actionnement (20), au moyen duquel le rotor de pompe (10) peut être actionné de manière hydraulique et/ou pneumatique, le rotor de pompe (10) comportant au moins un galet de rotor (30), qui peut être avancé et reculé au moyen du moyen d'actionnement (20), une force de pression et/ou de compression pouvant être générée sur un tuyau inséré dans la pompe péristaltique par le déplacement hydraulique et/ou pneumatique du rotor de pompe (10) au moyen du galet de rotor (30),
**caractérisé en ce que**,
le moyen d'actionnement (20) est ou comprend un vérin hydraulique et/ou pneumatique (22) ou piston hydraulique et/ou pneumatique (22) à double effet, qui, au moyen d'une surpression générée de manière hydraulique et/ou pneumatique, peut être déplacé activement dans une première et une seconde direction.

2. Rotor de pompe (10) selon la revendication 1, **caractérisé en ce qu'**au moins un moyen de commande et/ou de régulation est prévu et **en ce que** la force de pression et/ou de compression peut être commandée et/ou régulée de manière variable et/ou de manière commandée par le processus et/ou régulée par le processus au moyen du moyen de commande et/ou de régulation, en particulier en tenant compte des facteurs que sont le tuyau utilisé dans la pompe péristaltique, le type de fonctionnement et d'utilisation de la pompe péristaltique et/ou les composants utilisés tels qu'un filtre de dialyse en guise d'élément faisant partie d'un circuit de sang extracorporel, qui fonctionne avec la pompe péristaltique.

3. Pompe péristaltique comprenant au moins un rotor de pompe (10) selon la revendication 1 ou 2.

4. Machine de traitement du sang comprenant au moins un rotor de pompe (10) selon la revendication 1 ou 2 et/ou comprenant au moins une pompe péristaltique selon la revendication 3, la machine de traitement du sang étant de préférence une machine de dialyse.

5. Procédé d'actionnement d'un rotor de pompe (10) d'une pompe péristaltique, en particulier pour le réglage de la pression d'occlusion et/ou de la position des galets de rotor de la pompe péristaltique, le rotor de pompe (10) étant actionné de manière hydraulique et/ou pneumatique, le rotor de pompe (10) comportant au moins un galet de rotor (30), qui peut être avancé et/ou reculé de manière hydraulique et/ou pneumatique, une force de pression et/ou de compression étant générée sur un tuyau inséré dans la pompe péristaltique par le déplacement hydraulique et/ou pneumatique du rotor de pompe (10) au moyen du galet de rotor (30),
**caractérisé en ce que**,
le déplacement hydraulique et/ou pneumatique du rotor de pompe (10) dans une première et une seconde direction est effectué activement au moyen d'une surpression générée de manière hydraulique et/ou pneumatique et d'un vérin hydraulique et/ou pneumatique (22) et/ou piston hydraulique et/ou pneumatique (22) à double effet.

6. Procédé selon la revendication 5, **caractérisé en ce que** la force de pression et/ou de compression est commandée et/ou régulée de manière variable et/ou de manière commandée par le processus et/ou régulée par le processus, en particulier en tenant compte du tuyau utilisé dans la pompe péristaltique, du type de fonctionnement et d'utilisation de la pompe péristaltique et/ou des composants utilisés tels qu'un filtre de dialyse en guise d'élément faisant partie d'un circuit de sang extracorporel, qui fonctionne avec la pompe péristaltique.

7. Procédé selon l'une des revendications 5 ou 6, **caractérisé en ce que** le procédé est réalisé en utilisant au moins un rotor de pompe (10) selon l'une des revendications 1 ou 2, et/ou au moins une pompe péristaltique selon la revendication 3 et/ou au moins une machine de traitement du sang selon la revendication 4.

8. Utilisation d'un actionneur pneumatique et/ou hydraulique (22) pour le réglage de la pression d'occlusion d'une pompe péristaltique, la pompe péristaltique étant une pompe péristaltique avec les caractéristiques de la revendication 3.
